# FASCICULE DE BREVET EUROPEEN

(11) **EP 2 247 559 B1**
(45) Date de publication et mention de la délivrance du brevet: **31.07.2013**
(21) Numéro de dépôt: 08872860.5
(22) Date de dépôt: 16.12.2008
(51) Int. Cl.: C07C 7/144, C07C 11/06, C07C 9/08, C07C 7/04, B01D 3/14, B01D 61/36, C08F 10/06

(54) **PROCEDE DE SEPARATION DU PROPANE ET DU PROPYLENE METTANT EN OEUVRE UNE COLONNE A DISTILLER ET UNE UNITE DESEPARATION PAR MEMBRANE**
PROPAN/PROPYLEN-TRENNVERFAHREN MIT EINER DESTILLATIONSSÄULE UND EINER MEMBRANENTRENNEINHEIT
PROPANE/PROPYLENE SEPARATION PROCESS EMPLOYING A DISTILLATION COLUMN AND A MEMBRANE SEPARATION UNIT

(30) Priorité: 28.01.2008 FR 0800436
(43) Date de publication de la demande: 10.11.2010
(73) Titulaire: IFP Energies nouvelles, 92852 Rueil-Malmaison Cedex (FR)
(72) Inventeur: COUGARD, Nathalie, F-69005 Lyon (FR); BAUDOT, Arnaud, F-69390 Vernaison (FR); COUPARD, Vincent, FR-69120 Vaulx en Velin (FR)
(86) Numéro de dépôt international: PCT/FR2008/001746
(87) Numéro de publication internationale: WO 2009/106706

(56) Documents cités:
- WO-A-2008/084415
- US-A1- 2003 233 934
- US-A1- 2004 000 513
- US-A1- 2004 173 529
- US-A1- 2004 182 786

## Description

### DOMAINE DE L'INVENTION

Dans un contexte économique marqué par une demande croissante en propylène haute pureté (c'est à dire supérieure à 99,5 % molaire en propylène), utilisable dans les procédés de polymérisation, il est nécessaire, soit de développer de nouveaux moyens de production de propylène, soit d'améliorer les solutions existantes. La présente invention peut être considérée comme une amélioration des procédés existants basés sur la distillation seule en faisant appel à une combinaison d'unités de séparation par membrane et de colonnes de distillation que nous appelons dans la suite du texte procédé "hybride".

Une des étapes limitantes des procédés "hybrides" réside dans la séparation du propane et du propylène par la colonne à distiller. En effet, cette colonne est dimensionnée pour une certaine capacité et constitue généralement le goulot d'étranglement parmi les unités de fractionnement existantes dans les raffmeries.

En effet, les colonnes de séparation par distillation du propane et du propylène possèdent généralement plus de 150 plateaux théoriques et opèrent avec des taux de reflux très élevés (généralement compris entre 10 et 15), ce qui a pour conséquence directe des consommations énergétiques très importantes.

Outre l'aspect économique, il est important de noter également l'impact environnemental des émissions de CO₂ produites par les dites colonnes.

Il y a donc actuellement un enjeu décisif, aussi bien économique qu' environnemental, dans l'amélioration de la séparation du propane et du propylène.

La présente invention décrit un procédé permettant de réduire le coût énergétique de la séparation propane propylène, et de faire tomber ou de déplacer vers des valeurs plus élevées la limite d'engorgement des colonnes de distillation actuellement en place.

Le procédé objet de la présente invention est basé sur un couplage des unités de séparation par membranes avec la ou les colonnes à distiller permettant la séparation du propane et du propylène, ainsi que sur le choix des conditions opératoires desdites unités de séparation par membrane qui permet de réduire les coûts de recompression du perméat.

### EXAMEN DE L'ART ANTERIEUR

Les techniques membranaires sont souvent envisagées comme une alternative dans la séparation des couples oléfines/paraffines. Les séparation par membranes permettent de réduire les coûts d'opérations de manière notable comparativement à la distillation classiquement employée.

Les membranes de séparation connaissent déjà certaines applications industrielles.

Parmi celles ci on peut citer:
- la séparation par membrane de pervaporation, notamment dans le domaine de la déshydratation de solvant. Cette technique est appliquée notamment pour la purification de l'éthanol (couplage distillation - pervaporation), de l'alcool isopropylique, de l'acétone et de l'acide acétique. La pervaporation est également utilisée dans l'élimination des composés organiques volatiles des effluents aqueux. L'utilisation de membranes caoutchouteuses hydrophobes permet d'éliminer les traces de composants organiques dans l'eau. Dans ce cas, la séparation est basée sur les différences de solubilités entre la charge liquide et le matériau membranaire, elle est donc très efficace pour des composés tels que le chloroforme et les hydrocarbures aromatiques. Enfin, la pervaporation est aussi utilisée pour la séparation de mélange organique. Dans ce cas particulier, la pervaporation peut éventuellement se substituer (ou s'associer) à la distillation. Elle peut être utilisée notamment dans le cadre de la production d'éther carburant.
- Dans le cadre de la séparation oléfines/paraffines, il a été démontré que les membranes de séparation ne pouvaient pas être économiquement viables lorsqu'elles sont utilisées seules "Separation of Ethylene form Ethane using Perfluorosulfonic Acid Ion Exchange Membranes" (de Sungpet et al., ACS Symposium Series "Chemical Separation with Liquid Membranes" 270-285, 1996. [2] qu'on peut traduire par "Séparation de l'éthylène et de l'éthane utilisant une membrane échangeuse d'ions à l'acide perfluorosulfonique".

Les travaux de Petterson et al. (Separation Technology 6, (1996), 175-187) se sont focalisés sur le couplage membrane de séparation avec une colonne à distiller. La colonne à distiller de référence appelée aussi "splitter" dans la terminologie anglo saxonne, est constitué de 152 plateaux théoriques et fonctionne initialement à un reflux de 24,1 pour séparer le propane/propylène jusqu'à obtention d'une pureté de 99,5%. En ce qui concerne le matériau membranaire, les auteurs évoquent l'utilisation d'un matériau type Nafion de sélectivité fixée. Dans leurs travaux, les membranes (fonctionnant en perméation gazeuse) sont essentiellement positionnées en parallèle de la colonne, celles-ci sont alimentées par un flux à la pression de la colonne (17,5 bar), le perméat étant à 3,5 bar, ce qui nécessite l'emploi d'un compresseur pour réinjecter le flux dans la colonne. Les auteurs affirment obtenir une capacité de traitement accrue grâce à l'addition de membrane, ainsi qu'une éventuelle réduction du trafic interne, et du reflux de la colonne. L'évaluation économique n'a pas été poursuivi jusqu'a son terme, les coûts de recompression du perméat recyclé dans la colonne n'ayant pas été pris en compte.
- Kookos dans Industrial and Engineering Chemistry Research 42 (2003) 1731-1738, qu'on peut traduire par "Recherche en génie chimique industriel" a également mené des recherches sur ce sujet. Ses travaux ont essentiellement porté sur des configurations de membranes (fonctionnant en perméation gazeuse) en parallèle de la colonne à distiller. Le procédé décrit par Kookos traite une charge à 70/30% molaire de propylène/propane, et vise des spécifications à 99% molaire en propylène en tête de colonne. Cette étude s'appuie sur une membrane de type carbone ayant une perméabilité de 390 barrer pour une sélectivité propylène/propane de 18,4. Le couple perméabilité/sélectivité est donc très élevé, mais la membrane type carbone est très fragile. Les auteurs envisagent l'aspect économique dans le cas de la construction d'unité nouvelle, mais pas dans un objectif de dégoulottage. De plus, en raison des conditions opératoires décrites (pression dans le perméat relativement faible), le procédé fait appel à un compresseur pour réinjecter le perméat en phase gazeuse dans la colonne.
- Le brevet US 2004/0173529 A1 décrit plusieurs schémas de séparation couplant les membranes de séparation à une colonne à distiller. Les différentes configurations envisagées (en amont et en aval de la membrane) dans le brevet cité comprennent des compresseurs, ce qui implique des coûts d'investissements importants. Ces compresseurs sont en effet nécessaires pour comprimer le flux du perméat se trouvant à pression atmosphérique, voir sous vide.

Les exemples utilisés dans ce brevet font état d'une colonne à distiller de 58 plateaux théoriques fonctionnant avec un taux de reflux de 53 pour obtenir un propylène à 99% molaire en sortie. Ce taux de reflux est très supérieur aux valeurs rencontrées industriellement. Par ailleurs, le procédé décrit dans le brevet cité est destiné à la purification du propylène obtenu par déshydrogénation du propane, ce qui n'est pas le cas dans la présente invention.

Le brevet US 6,899,743 B2 décrit un procédé hybride impliquant une combinaison de membranes et d'un déflegmateur. Ce procédé permet d'obtenir des séparations quatre à six fois plus efficaces qu'une simple condensation partielle. Ce procédé peut être utilisé dans différents domaines tels que la séparation d'un azéotrope, l'amélioration de performance d'une membrane seule dans le cas d'une charge monophasique en vue ou non d'être injecté dans une colonne à distiller. Ce brevet traite essentiellement de l'efficacité de la membrane permsélective. Les membranes en question sont efficaces dans la séparation des mélanges de composés aromatiques/aliphatiques, d'azéotropes (en particulier les mélanges contenant des alcools légers), de mélanges d'isomères d'hydrocarbures légers, et également d'hydrocarbures légers oléfines/paraffines (notamment le propane/propylène). Le matériau décrit assure une perméabilité de 40 à 50 GPU (environ 40 à 50 barrer) pour une épaisseur sélective de membrane de 1µm et pour une sélectivité moyenne du propylène sur le propane de 4,5. Dans ce brevet, les auteurs n'explicitent pas les conditions opératoires ciblées. Le brevet explicite tout de même le fait que le perméat est maintenu sous vide à l'aide d'une pompe à vide. Le procédé du brevet cité est différent de la présente invention notamment par le fait que les auteurs ajoutent une étape de déflegmation.

Enfin, US 2004000513 et le brevet US 7,070,694 B2 traitent également de l'intégration des membranes "perm-sélectives" dans les procédés de séparation par distillation de mélanges organiques. Les performances des procédés ont été évaluées avec des membranes possédant une très forte sélectivité propane/propylène (environ 15) et une perméabilité réduite (2 barrer).

Ces membranes sont opérées en perméation gazeuse et placées en tête de la colonne à distiller ou en parallèle. La faible pression en sortie de la membrane (environ 40 psia, soit 2,75 bar selon les exemples) impose de comprimer le perméat afm de récupérer le propylène PG (99,5% molaire en propylène), d'où des coûts de recompression vraisemblablement élevés.

De manière générale, les procédés de séparation membranaire décrit dans la littérature nécessitent un perméat à basse pression (proche de la pression atmosphérique, voire sous vide) de manière à bénéficier de la plus grande différence de pression possible de part et d'autre de la membrane. Dans ce cas, afm de pouvoir réinjecter le perméat dans le procédé, il est nécessaire soit de le condenser avec un groupe frigorifique (opération très coûteuse en investissement et coûts opératoires), soit de le recomprimer (opération également très coûteuse).

Le présent procédé, à la différence des procédés décrit dans l'art antérieur, utilise une pression partielle de propylène dans la charge proche du point critique en amont, pour disposer ensuite d'un flux de perméat à une pression telle qu'il puisse être directement réinjecté aisément dans la colonne à distiller de séparation propane/propylène à une pression proche de la pression du plateau d'injection de ladite colonne. Si la pression du perméat est égale à celle du plateau d'injection, le perméat peut être injecté directement en phase gaz dans la colonne. Si la pression du perméat est seulement proche (par exemple à plus ou moins 3 bars) de celle du plateau de réinjection, on réalisera alors une condensation (à eau ou à air), puis une remise en pression par pompe, procédé très peu coûteux.

Un des avantages significatifs d'utiliser le module membranaire à des pressions et température plus importantes que celles pratiquées selon l'art antérieur, réside dans le fait que le présent procédé ne nécessite pas de compression du perméat gazeux, contrairement aux autres procédé décrit dans l'art antérieur.

Globalement, le procédé selon l'invention est donc nettement plus économique que les procédés de l'art antérieur.

De plus, la réduction du coût énergétique de la colonne à distiller s'accompagne également d'un impact favorable sur l'environnement en raison de la diminution des rejets de gaz à effets de serre (GES), notamment du CO2.

### DESCRIPTION SOMMAIRE DES FIGURES ET DEFINITION DES TERMES

Dans la suite du texte on parle de module membranaire pour désigner la membrane seule (ensemble de fibres creuses recouvertes de polymère et regroupés dans la même calandre), et d'unité de séparation membranaire pour désigner un groupe de module membranaire, ledit groupe pouvant comporter une, deux, trois ou davantage de modules membranaire fonctionnant en série ainsi que l'appareillage nécessaire au bon fonctionnement de ladite unité (échangeur, pompe).

On parle de "conditions de pervaporation", lorsque la pression prise en entrée de chaque module membranaire sur la face amont de la membrane (en contact avec la charge à séparer) est comprise entre 20 bar et 47 bar (1 bar = 10⁵ pascal), et lorsque la température de charge est comprise entre 50°C et 92°C.

On parle de "conditions supercritiques" lorsque la pression prise en entrée de chaque module membranaire sur la face amont de la membrane (en contact avec la charge à séparer) est comprise entre 47 bar et 100 bar, et lorsque la température de charge est comprise entre 92°C et 200°C.
La figure 1 est une représentation d'un schéma de procédé selon l'invention, comportant une unité de séparation membranaire située en amont de la colonne à distiller.
La figure 2 est une représentation d'un schéma de procédé selon l'invention, comportant une unité de séparation membranaire située en aval de la colonne à distiller.
La figure 3 est une représentation d'un schéma de procédé selon l'invention comportant à la fois une unité de séparation membranaire en amont de la colonne, et une unité de séparation membranaire en aval de ladite colonne.
La figure 4 représente une unité de séparation membranaire, possédant un seul module membranaire, et travaillant en conditions de pervaporation.

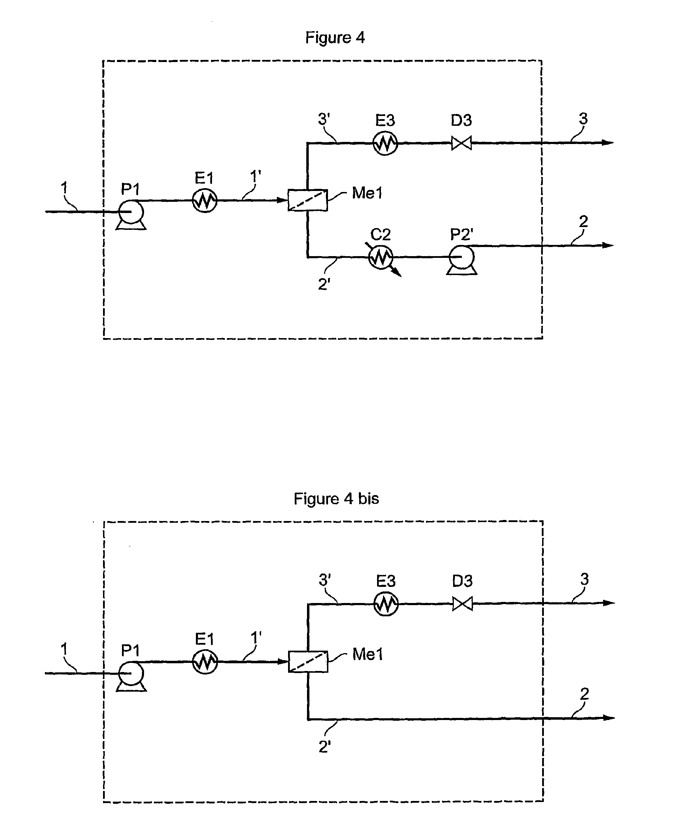

La figure 4 bis représente une unité de séparation membranaire, possédant un seul module membranaire, et travaillant en conditions de pervaporation avec une pression du perméat égale à (ou très proche de) la pression du plateau de réintroduction dans la colonne.

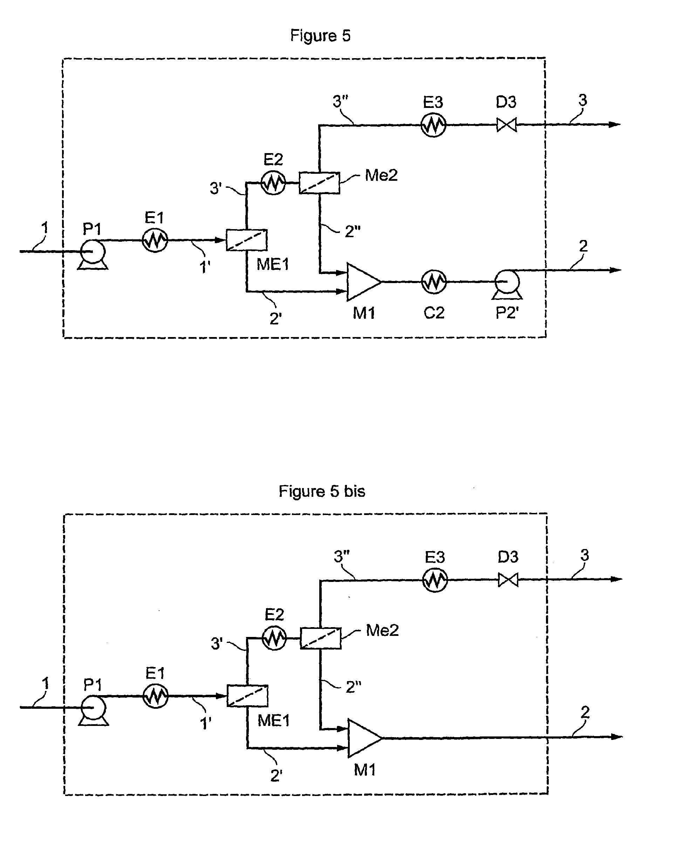

La figure 5 représente une unité de séparation membranaire, possédant deux modules membranaires travaillant en série, et en conditions de pervaporation.
La figure 5 bis représente une unité de séparation membranaire, possédant deux modules membranaires travaillant en série, et en conditions de pervaporation avec une pression du perméat égale à (ou très proche de) la pression du plateau de réintroduction dans la colonne.

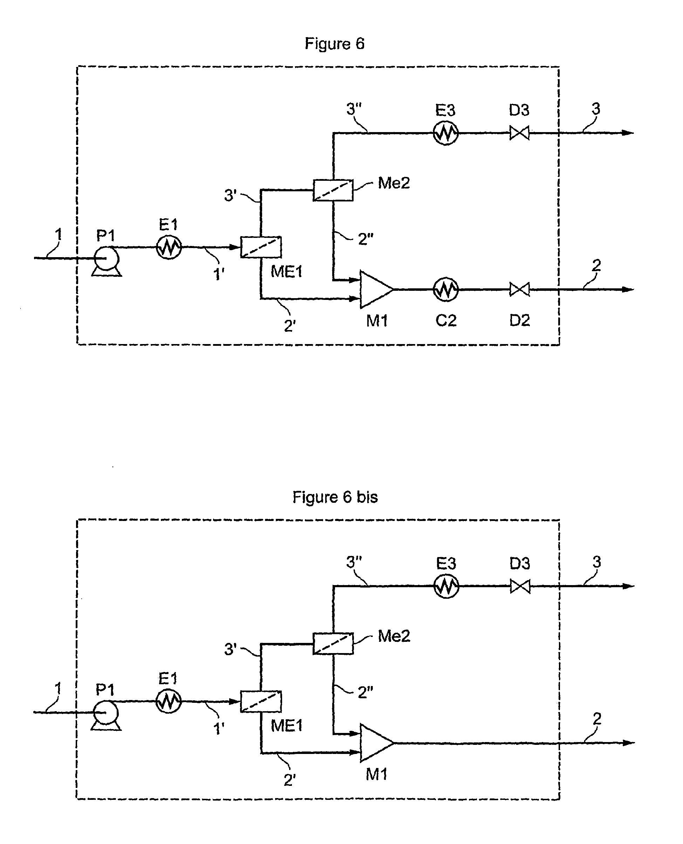

La figure 6 représente une unité de séparation membranaire, possédant deux modules membranaires travaillant en série et en conditions supercritiques.
la figure 6 bis représente une unité de séparation membranaire, possédant deux modules membranaires travaillant en série et en conditions supercritiques avec une pression du perméat égale à (ou très proche de) la pression du plateau de réintroduction dans la colonne.

### DESCRIPTION SOMMAIRE DE L'INVENTION

La présente invention peut se définir comme un procédé de séparation du propane et du propylène mettant en oeuvre une colonne à distiller et au moins une unité de séparation membranaire constituée de plusieurs modules membranaire travaillant en série, ladite unité de séparation membranaire étant placée soit en amont, soit en aval, soit en amont et en aval de la colonne à distiller, la ou lesdites unités de séparation membranaires fonctionnant avec des membranes polymères de perméabilité supérieures à 0,1 barrer et de sélectivité propane/propylène supérieure à 5,
- soit aux conditions de pervaporation suivantes, prises en entrée de chaque module membranaire: 20 bar < pression < 47 bar et 50°C < température < 92°C,
- soit aux conditions supercritiques suivantes, prises en entrée de chaque module membranaire: 47< pression<100 bar et 92°C<température <200°C.

Lorsque le procédé selon l'invention fonctionne en conditions de pervaporation prises en entrée de chaque module membranaire (pression de charge comprise entre 20 et 47 bar et température de charge entre 20°C et 92°C), la ou les dites unités de séparation fonctionnent avec une différence de pression telle que le perméat soit à une pression d'au minimum 10 bar.

Dans ce cas, le perméat peut être:
a) réinjecté directement dans la colonne en phase vapeur ou
b) directement valorisé en tant que propylène haute pureté.

En conditions de fonctionnement de pervaporation, et lorsque la pression du perméat est égale ou supérieure à la pression de la colonne, ce dernier peut être réintroduit directement dans la dite colonne, moyennant éventuellement une détente ou une condensation (cas b).

Lorsque le procédé fonctionne en conditions supercritiques prises en entrée de chaque module membranaire (pression de charge comprise entre 47 et 100 bar, et température de charge entre 92°C et 200°C), la ou les dites unités de séparation fonctionnent avec une différence de pression telle que le perméat soit à une pression d'au minimum de 10 bar.

Dans ce cas, le perméat peut être:
a) réinjecté directement dans la colonne en phase vapeur
b) condensé, repompé puis réintroduit en phase liquide dans la colonne.
c) valorisé en tant que propylène haute pureté utilisable dans les procédés de polymérisation.

En conditions de fonctionnement supercritiques, et lorsque la pression du perméat est supérieure ou égale à la pression de la colonne, ce dernier peut être réintroduit directement dans la colonne, moyennant éventuellement une détente ou une condensation (cas c).

### DESCRIPTION DETAILLEE DE L'INVENTION

La présente invention est un procédé de séparation du propane et du propylène mettant en oeuvre une colonne de distillation et au moins une unité de séparation membranaire placée en aval, en amont ou encore en aval et en amont de ladite colonne, ledit procédé permettant de diminuer la charge de la colonne de distillation propane propylène, et ce, sans ajouter de compresseur, ce qui est un point essentiel dans l'économie du procédé.

Le procédé peut éventuellement mettre en oeuvre plusieurs colonnes à distiller, la présente invention pouvant s'appliquer à l'une ou plusieurs de ces colonnes.

De manière générale, c'est à dire pour toutes les configurations correspondant au procédé selon l'invention, le flux de charge à traiter est une coupe C3 de raffinerie. Ce flux contient majoritairement du propane et du propylène.

Ce flux peut contenir en outre des impuretés tels que le COS, H2S, des mercaptans et des diènes. De manière générale, la teneur en propylène de la charge à traiter est au minimum de 60% poids.

Le procédé selon l'invention peut par exemple être appliqué dans le cadre du dégoulottage d'un gaz plant de FCC (abréviation de craquage catalytique en lit fluidisé). En effet, la ou les colonnes de séparation propane/propylène constituent très souvent un goulot d'étranglement pour la production de propylène.

Or l'intégration d'unités de séparation membranaire en amont, en aval, ou en amont et en aval de la colonne de distillation augmente la capacité de traitement de la dite colonne d'environ 30%, comme cela est montré dans l'exemple faisant partie de la présente demande.

Ces unités de séparation par membranes, placées selon diverses configurations autour de la colonne de distillation propane/propylène, permettent de concentrer en propylène le flux traversant la membrane appelé perméat, et par voie de conséquence d'en appauvrir le flux résiduel appelé rétentat. Ces unités peuvent fonctionner de manière générale avec un flux de charge contenant au minimum 60% massique en propylène. Dans le cas des unités de séparation dont le perméat est réintroduit dans la colonne (en amont ou en aval), la charge contient de manière préférée 70 % massique en propylène, et dans le cas d'une unité de séparation située en aval de la colonne à distiller, on utilisera préférentiellement une charge contenant un minimum de 90% massique de propylène.

Le transport des espèces à séparer à travers la membrane est assuré en maintenant la pression partielle dans le perméat plus faible que dans le rétentat. Cette faible pression partielle du perméat peut être assurée par divers moyens tels que la condensation du perméat. En vue d'atteindre le niveau de pression voulu dans le perméat dans le cadre de cette invention, la condensation du flux du perméat constitue l'option la plus courante. Néanmoins, toute autre solution permettant de réaliser un faible niveau de pression du perméat reste dans le cadre de la présente invention.

Les deux flux de rétentat et de perméat sont ensuite réinjectés dans la colonne de distillation propane/propylène à des niveaux de ladite colonne qui, de préférence, correspondent au mieux aux compositions desdits rétentat et perméat.

Une partie des schémas de procédé selon la présente invention repose sur la technique de la pervaporation. La force motrice mise en jeu dans la pervaporation provient de la différence de pression partielle de part et d'autre de la membrane. Les flux entrent dans la membrane à l'état liquide, sont évaporés à travers la paroi membranaire et ressortent à l'état gaz.

L'efficacité des membranes est définie en terme de sélectivité (rapport de la perméabilité du propylène sur celle du propane) et de perméabilité (caractéristique de la membrane définissant la diffusivité de la charge à traiter à travers ladite membrane).

Les modules membranaires intervenant dans les unités de séparation membranaire du procédé selon l'invention fonctionnent sur le principe de la pervaporation ou de la perméation gazeuse, ceci pour des conditions de température et de pression couvrant un large domaine d'utilisation. Typiquement entre 20°C et +200°C, et préférentiellement entre 50°C et 100 °C, pour des pressions de charge comprises entre 20 bar absolu et 100 bar (1 bar =10⁵ pascal).

Le polymère constituant la membrane est du type organique. Celui-ci présente une sélectivité propane/propylène d'au moins 5, et préférentiellement d'au moins 10, et une perméabilité au minimum de 0,1 barrer (sélectivité et perméabilité étant rapportées au couple propane/propylène). Le barrer est une unité spécifique aux technologies de séparation par diffusion à travers des films denses, et équivaut à 10⁻¹⁰ Ncm³.cm/cm⁻².cm_{Hg}⁻¹.s⁻¹
- Dans le cas où les conditions opératoires des unités de séparation par membrane sont choisies inférieures au point critique (P < 47 bar et T < 92°C), la pression de perméat est choisie égale ou proche de la pression de la colonne à distiller, c'est à dire supérieure ou égale à 15 bar. La température fixée dans le module membranaire est donc choisie de manière que a) la charge soit en phase liquide, b) la pression partielle du propylène dans la charge soit toujours supérieure à la pression partielle du propylène dans le perméat, et c) que le perméat soit toujours en phase gaz.

Le flux de charge devant être réchauffé pour permettre l'évaporation du perméat au cours de l'étape de pervaporation, il en résulte la présence d'un réchauffeur intermédiaire entre chaque module membranaire, comme cela est montré dans les figures 5 et 5bis.
- Dans le cas où les conditions opératoires sont choisies en milieu supercritique (P > 47 bar et T>92°C), la charge en amont de la membrane est dans des conditions supercritiques, alors que le perméat est en phase vapeur. La membrane n'est alors plus le siège d'une évaporation puisqu'en conditions supercritiques, il n'y a pas de changement d'état lorsque l'on passe du domaine supercritique à la phase vapeur. Si l'on compare cette situation avec celle de pervaporation, il n'y a donc plus dans le rétentat de perte de chaleur induite par la vaporisation du perméat. Ceci implique qu'il n'y a plus besoin de réchauffeur intermédiaire entre les différents modules membranaires, comme cela est montré sur les figure 6 et 6 bis. De la même manière que précédemment, la pression de perméat est choisie égale ou proche à la pression de la colonne à distiller c'est à dire supérieure ou égale à 15 bar.

De manière générale, c'est à dire pour toutes les configurations correspondant au procédé selon l'invention, Le flux de charge à traiter est une coupe C3 de raffinerie. Ce flux contient majoritairement du propane et du propylène.

Ce flux peut contenir en outre des impuretés tels que le COS, H2S, des mercaptans et des diènes. La teneur en propylène d'une telle charge est d'un minimum de 60% poids.

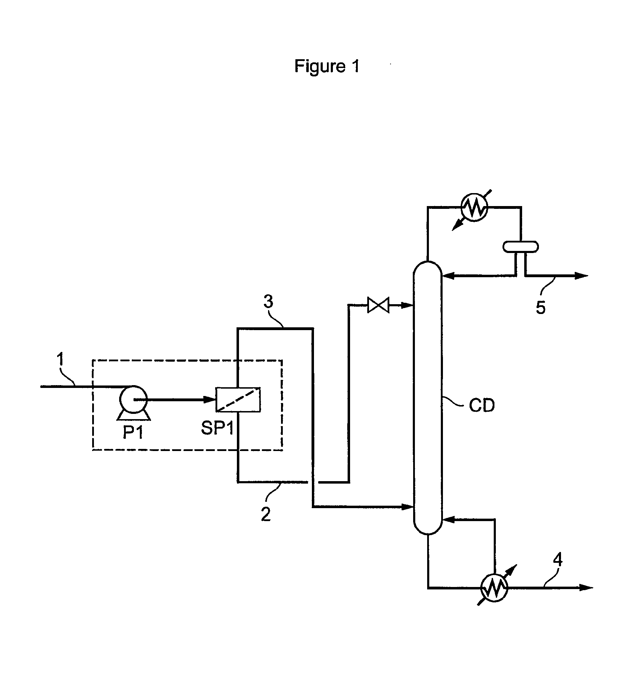

La suite de la description sera mieux comprise au moyen des figures 1 à 6 décrites ci dessous.

### Figure 1

La première configuration du procédé, dite "cas amont", est représentée de manière schématique par la figure 1. On utilise une unité de séparation membranaire (SP1) possédant un ou plusieurs modules membranaires placés en amont de la colonne à distiller (CD), ladite unité de séparation membranaire fonctionnant en condition de pervaporation ou en conditions supercritiques.

La charge à séparer (1) est introduite dans l'unité de séparation membranaire (SP1), de laquelle on extrait un perméat vapeur (2) et un rétentat liquide (3).

Le perméat vapeur (2) est réintroduit dans la partie supérieure de la colonne (CD).

Le rétentat liquide (3) est réintroduit dans la partie inférieure de la colonne (CD).

On extrait en tête de la colonne (CD) un flux (5) de propylène à haute pureté, et en fond de colonne un flux (4) de propane.

### Figure 2

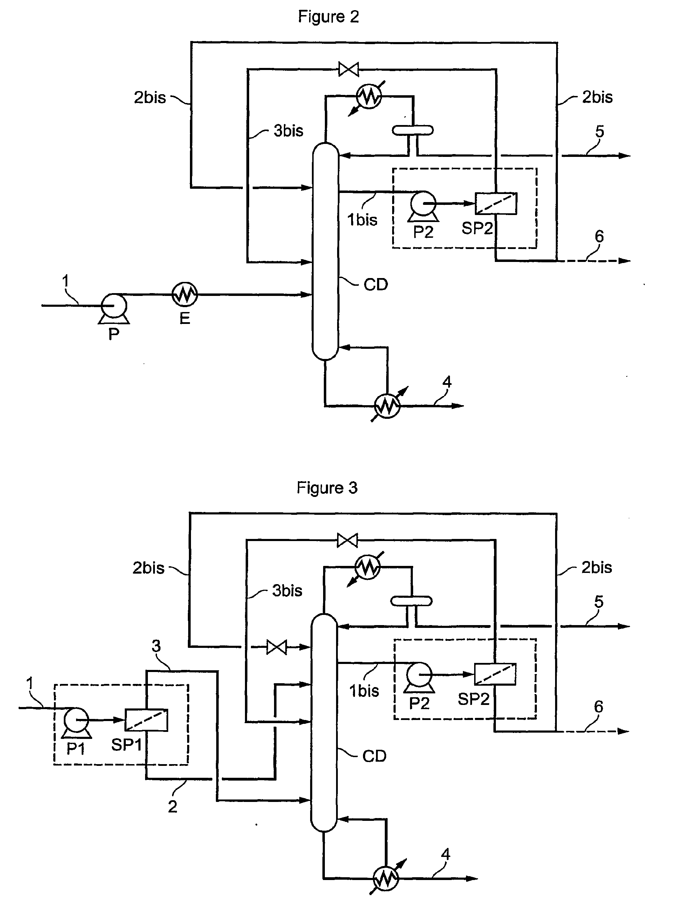

La seconde configuration du procédé selon l'invention, dite "cas aval", est représentée par la figure 2. On utilise une unité de séparation membranaire (SP2) possédant un ou plusieurs modules membranaires fonctionnant en série, ladite unité de séparation membranaire étant placée en aval de la colonne (CD) et pouvant fonctionner en conditions de pervaporation ou en conditions supercritiques.

La charge à séparer (1) est introduite dans la colonne (CD), éventuellement après pompage au moyen d'une pompe (P), et réchauffage au moyen d'un échangeur (E) pour se retrouver dans des conditions de pression et température voisines de celles existantes au point d'introduction dans ladite colonne.

Le flux (1 bis) est soutiré de la colonne (CD) et est introduit dans l'unité de séparation membranaire (SP2) après passage dans la pompe P2 pour atteindre le niveau de pression souhaité. On extrait de l'unité de séparation membranaire (SP2) un rétentat (3bis) qui est renvoyé dans la partie inférieure de la colonne (CD), et un perméat (2bis) qui est a) soit réintroduit dans la partie supérieure de la colonne, b) soit directement valorisé comme propylène haute pureté par le flux (6).

Le choix entre les deux possibilités a) et b) dépend essentiellement du point de soutirage du flux alimentant l'unité de séparation membranaire (SP2).

### Figure 3

La troisième configuration du procédé selon l'invention, dite cas "amont+aval", est représentée par la figure 3. On utilise deux unités de séparation membranaires (SP1) et (SP2), l'une (SP1) étant placée en amont, l'autre (SP2) étant placée en aval de la colonne (CD).

Cette configuration peut être décrite comme la superposition du cas "amont" et du cas "aval" précédemment décrit. Il convient de noter que dans cette configuration "amont +aval", l'unité de séparation amont peut fonctionner en conditions de pervaporation, et l'unité de séparation aval peut fonctionner en conditions supercritiques.

Ou bien encore, l'unité de séparation amont peut fonctionner en conditions supercritiques, et l'unité de séparation aval peut fonctionner en conditions de pervaporation.

Enfin, les unités de séparation amont et aval peuvent fonctionner toutes les deux en conditions de pervaporation ou en conditions supercritiques.

Les flux ayant le même numéro sur les figures 1, 2 et 3 correspondent à des flux identiques.

### Figures 4 et 4 bis

Les figure 4 et 4 bis représentent une unité de séparation possédant un seul module membranaire ( Me1), ladite unité de séparation pouvant être placée en amont ou en aval de la colonne (CD non représentée sur les figures 4 et 4 bis).

La charge à traiter (1) est pompée au moyen de la pompe P1, puis réchauffée au moyen de l'échangeur (E1) jusqu'à une température approchant de moins de 10°C la température de bulle de la charge à la pression amont du module membranaire (Me1). La charge (1) devenant (1') est ensuite introduite dans le module membranaire (Me1) duquel on extrait un rétentat (3') et un perméat (2').

Le perméat (2') devenant (2) est:
a) soit condensé dans le condenseur (C2), puis pompée dans la pompe (P2'), puis réintroduit dans la colonne à distiller (CD) tel que représenté sur la figure 4,
b) soit réintroduit directement dans la colonne (CD) tel que représenté sur la figure 4 bis.

Le rétentat liquide (3') devenant (3) est réintroduit en fond de la colonne à distiller (CD) après refroidissement dans l'échangeur (E3) et détente dans le détendeur (D3).

La description reste la même, que le module membranaire fonctionne en conditions de pervaporation ou en conditions supercritiques.

### Figures 5 et 5 bis

Les figures 5 et 5 bis représentent le cas où l'unité de séparation possède deux modules membranaires fonctionnant en conditions de pervaporation.

La charge à traiter (1) est pompée au moyen de la pompe P1 puis réchauffée au moyen de l'échangeur (E1) jusqu'à une température approchant de moins de 10°C la température de bulle de la charge à la pression amont du module membranaire (Me1). La charge (1) est ensuite introduite dans le premier module membranaire (Me1) duquel on extrait un rétentat (3') et un perméat (2').

Le rétentat liquide (3') issu du premier module membranaire (Me1) passe à travers un échangeur (E2) pour être réchauffé jusqu'à une température approchant de moins de 10°C la température de bulle de ce rétentat, puis est réintroduit dans le second module membranaire (Me2).

Le perméat (2") issu du second module membranaire (Me2) est alors ajouté au perméat (2') issu du premier module membranaire (Me1) grâce au mélangeur (M1). Le perméat (2") est:
a) soit condensé dans le condenseur (C2) et réintroduit dans la colonne à distiller (CD), non représentée sur les figures 5 et 5 bis, par le flux (2) après passage dans une pompe (P2') tel que représenté figure 5,
b) soit réintroduit directement dans la colonne (CD) par le flux (2) tel que représenté figure 5 bis.

Le rétentat liquide (3") issu du second module membranaire (Me2) est réintroduit en fond de la colonne à distiller (CD) sous la forme du flux (3) après refroidissement dans l'échangeur (E3) et détente dans le détendeur (D3).

La description est la même que l'unité membranaire soit placée en amont ou en aval de la colonne (CD).

### Figures 6 et 6 bis

Les figures 6 et 6 bis représentent le cas où l'unité de séparation possède deux modules membranaires fonctionnant en conditions supercritiques.

La charge à traiter (1) est pompée au moyen de la pompe P1 puis réchauffée au moyen de l'échangeur (E1) jusqu'à une température supérieure à 92°C.

La charge (1) est ensuite introduite dans le premier module membranaire (Me1) duquel on extrait un rétentat liquide (3') et un perméat vapeur (2').

Le rétentat liquide (3') issu du premier module membranaire (Me1) est réintroduit directement dans le second module membranaire (Me2).

Le perméat (2"), devenant (2), issu du second module membranaire (Me2) est alors ajouté au perméat (2') issu du premier module membranaire (Me1) grâce au mélangeur (Ml). Le perméat (2") est
a) soit condensé dans le condenseur (C2) et réintroduit dans la colonne à distiller (CD), non représentée sur les figures 6 et 6 bis, par le flux (2), après passage dans une pompe (P2) tel que représenté figure 6,
b) soit réintroduit directement dans la colonne (CD) par le flux (2), tel que représenté figure 6 bis. Le rétentat liquide (3") devenant (3) issu du second module membranaire (Me2) est réintroduit en fond de la colonne à distiller (CD) après refroidissement dans l'échangeur (E3) et détente dans le détendeur (D3).

### EXEMPLES

Les trois exemples suivants illustrent l'intérêt de l'invention en termes de rentabilité économique et d'augmentation de la capacité de production.

Les 2 premiers exemples sont conçus sous la forme d'une comparaison entre 3 schémas selon l'invention, (notés cas "amont"/ cas "aval" / cas "amont+aval") et un cas de base correspondant à l'art antérieur et ne possédant qu'une colonne à distiller (sans aucune unité de séparation par membrane). Le troisième exemple selon l'invention, compare les conditions de fonctionnement en pervaporation et en supercritiques.

Dans tous les exemples selon l'invention, on utilise une membrane polymère de perméabilité égale à 10 barrer et de sélectivité du Propane/Propylène égale à 10.

### Exemple N°1 : Comparaison des performances du procédé selon l'invention / art antérieur dans les "conditions de pervaporation ")

Cet exemple permet d'évaluer le gain atteignable en intégrant les unités de séparations membranaire à la colonne de distillation propane/propylène dans l'objectif de traiter une même capacité de charge.

### • Cas de base (art antérieur)

Le cas de base consiste en une séparation d'une charge propane/propylène réalisée au moyen d'une colonne à distiller comportant 170 plateaux et travaillant avec un taux de reflux de 13.

**Tableau 1: Caractéristique de la colonne à distiller Propane/Propylène utilisée pour le cas de base**

| | |
|---|---|
| Temperature tête | 49,1 °C |
| Temperature fond | 65,8 °C |
| Pression tête | 20,5 bars |
| Plateaux théoriques | 168 + condenseur & rebouilleur |
| Alimentation | liquide au plateau 121 |
| Soutirage tête | 99,5 % mass. de propylène |
| Soutirage fond | 5 % mass. de propylène |
| ΔP | 0,01 bar/plateau |

Le coût de fonctionnement principal de la colonne de distillation se trouve dans la chaleur à fournir au rebouilleur et au condenseur.

Le tableau 1 ci dessous donne la composition de la charge à traiter, les conditions opératoires, et les spécifications sur les produits fixées comme objectifs.

**Tableau 2 : bilan matière du cas de base**

| | | CHARGE | PROPANE | PROPYLENE |
|---|---|---|---|---|
| Description des flux | | | | |
| Phase | | Liquide | Liquide | Liquide |
| Pression | bar | 25,38 | 22 | 20,1 |
| Température | °C | 63,1 | 69 | 49 |
| Débit massique | kg/h | 16699,8 | 4651,3 | 12048,5 |
| PROPENE | %masse | 73 | 5 | 99,5 |
| PROPANE | %masse | 20 | 72 | 0,5 |
| BUTANE | %masse | 7 | 23 | 0,0 |

Le tableau 3 donne les chaleurs échangées au rebouilleur et au condenseur de la colonne de distillation pour le cas de base.

**Tableau 3 : Chaleurs au condenseur et au rebouilleur de la colonne à distiller dans le cas de base**

| Appareillage | Chaleur échangée [MW] |
|---|---|
| Condenseur | 18,3 |
| Rebouilleur (type BKT) | 18,2 |

### • Cas amont (selon l'invention)

La Figure 1 représente le schéma du procédé selon l'invention selon le cas amont dans lequel l'unité de séparation (SP1) est placée en amont de la colonne (CD).

La charge est initialement pompée et réchauffée par la pompe (P1) et l'échangeur (E1).

L'unité de séparation (SP1) fonctionne en conditions de pervaporation.

L'unité de séparation (SP1) à un module (Me1) est décrite à la Figure 4.

La charge est portée à 40 bar et 83°C (4°C en dessous du point de bulle).

La différence de pression à travers la membrane est un paramètre à définir selon deux possibilités :
- ΔP = 20 bar, le perméat est à la même pression que le plateau d'injection, ce qui permet de réinjecter le perméat directement dans la colonne. Ce n'est pas le choix fait dans cet exemple.
- ΔP = 25 bar (configuration choisie dans cet exemple), la différence de pression est maximisée pour obtenir plus d'efficacité de séparation. Le perméat se trouve alors à une pression inférieure à celle de la colonne, on place donc un condenseur sur le flux du perméat suivi d'une pompe avant de le réinjecter (ici T _{condensation} = 33°C, utilisation d'un condenseur à eau).

Le perméat [2] est réintroduit entre les plateaux 1 et 85, précisément dans cet exemple au plateau 76. Le rétentat [3] est réintroduit entre les plateaux 170 et 86, précisément dans cet exemple au plateau 133.

Les conditions de fonctionnement choisies pour l'exemple sont les suivantes :

**Tableau 4 : Configuration utilisée pour le calcul de l'exemple "cas amont"**

| | |
|---|---|
| P_{charge} | 40 bar |
| T_{charge} | 83,2°C |
| ΔP | 25 bar |
| Unité de séparation | une unité de séparation en amont incluant un module membranaire (cf. Figure 4) |
| réinjection du perméat dans la colonne | après condensation à 33°C |

Le tableau 5 ci dessous donne le bilan matière autour de l'unité de séparation

**Tableau 5 : bilan matière sur la membrane placée en amont de la colonne à distiller**

| Nom du flux | | CHARGE | PERMEAT | RETENTAT |
|---|---|---|---|---|
| Description | | [1] | [2] | [3] |
| Pressure | bar | 39,5 | 15 | 39 |
| Température | °C | 83,2 | 67,2 | 67,2 |
| Total Mass Rate | kg/h | 19300,00 | 6101,48 | 13198,52 |
| ETHANE | % poids | 0,000 | 0,001 | 0,000 |
| PROPENE | | 0,732 | 0,944 | 0,634 |
| PROPANE | | 0,204 | 0,055 | 0,272 |
| BUTANE | | 0,061 | 0,000 | 0,089 |
| 12BD | | 0,003 | 0,000 | 0,005 |

### • Cas aval (selon l'invention)

La figure 2 représente un schéma du procédé selon l'invention selon le cas aval dans lequel l'unité de séparation (SP2) est placée en aval de la colonne (CD). Comme indiqué sur la figure 2, le perméat (2) peut être soit réinjecté dans la colonne par le flux (2bis), soit sortit du procédé dans le cas ou celui-ci par le flux (6) possède déjà la pureté spécifiée.

La charge à traiter est soutirée à un plateau tel que la composition de la charge est supérieure à la composition en entrée de procédé, puis la charge est pompée et réchauffée au moyen de la pompe (P1) et de l'échangeur (E1).

On effectue le soutirage de liquide (1') à partir de la colonne au plateau 70.

Le liquide soutiré (1') est injecté dans l'unité de séparation membranaire (SP2) sous pression et température proche du point critique.

En sortie de l'unité de séparation membranaire aval (SP2), on réinjecte le perméat (2) dans la colonne (flux 2bis), au niveau du plateau 28, et on réintroduit le rétentat (3) au niveau du plateau 83.

Les configurations possible pour l'unité de séparation (SP2) à un module sont décrites à la Figure 4 et 4 bis.

Les conditions de fonctionnement choisies pour l'exemple sont les suivantes:

**Tableau 6 : Configuration utilisée pour le calcul de l'exemple "cas aval"**

| | |
|---|---|
| P_{charge} | 40 bar |
| T_{charge} | 79,4°C |
| ΔP | 25 bar |
| Unité de séparation | unité de séparation incluant un module membranaire |
| réinj ection du perméat dans la colonne | après condensation à 35°C (condenseur à eau) |

Le tableau 7 ci dessous donne le bilan matière autour de l'unité de séparation

**Tableau 7 : bilan matière sur la membrane placée en aval de la colonne à distiller**

| Nom du flux | | CHARGE | PERMEAT | RETENTAT |
|---|---|---|---|---|
| Description | | [1] | [2] | [3] |
| Pression | bar | 39,5 | 15 | 39 |
| Température | °C | 79,4 | 52,4 | 52,4 |
| Débits | kg/h | 13000,0 | 4694,5 | 8305,5 |
| ETHANE | % masse | 0,00 | 0,00 | 0,00 |
| PROPENE | | 0,89 | 0,99 | 0,84 |
| PROPANE | | 0,11 | 0,01 | 0,16 |
| BUTANE | | 0,00 | 0,00 | 0,00 |
| | | | | |

### • Cas amont + aval (selon l'invention)

La figure 3 représente un schéma du procédé selon l'invention dans le cas "amont + aval" qui fait appel à 2 unités membranaires, SP1 placé en amont de la colonne à distiller (CD), et SP2 placé en aval de ladite colonne.

L'unité de séparation membranaires (SP1) en aval, fonctionne aux mêmes conditions que dans le cas "aval".

L'unité de séparation membranaire (SP2) en amont, fonctionne aux mêmes conditions que dans le cas "amont".

Les conditions de fonctionnement sont données dans le tableau 8 ci dessous:

**Tableau 8 : Configuration utilisée pour le calcul de l'exemple cas "amont + aval"**

| | Unité en Amont (SP1) | Unité en Aval (SP2) |
|---|---|---|
| P_{charge} | 40 bar | 40 bar |
| T_{charge} | 83,2°C | 79,4°C |
| ΔP | 25 bar | 25 bar |
| Unité de séparation | une unité de séparation en amont incluant un module membranaire (cf. Figure 4) | une unité de séparation en aval incluant deux modules membranaire (cf. Figure 5) |
| réinjection du perméat dans la colonne | après condensation à 35°C (condenseur à eau) | après condensation à 35°C (condenseur à eau) |

Les trois cas "amont"/"aval"/"amont+aval" ont été comparés par simulation au moyen du logiciel commercial PROII pour un même débit de charge à séparer. Les procédés selon l'invention ("amont"/ "aval"/ "amont +aval") permettent une réduction de la chaleur échangée au rebouilleur de la colonne allant jusqu'à 20% pour le cas "amont+aval".

Le tableau 9 ci dessous présente les résultats de cette comparaison.

**Tableau 9 : Évaluation du Gain d'énergie pour les 3 schémas proposés**

| | Cas de Référence | Cas "amont" | Cas "aval" | Cas "amont+aval" |
|---|---|---|---|---|
| Débit de charge (kg/h) | 16 700 | | | |
| Q rebouilleur (MW) | 18,2 | 16,3 | 16,3 | 14,5 |
| % réduction /cas de base | - | 10,4% | 10,4% | 20,3% |

### Exemple N°2 illustrant l'augmentation de la capacité de la colonne à distiller dans les "conditions de pervaporation "

Le procédé selon l'invention permet d'augmenter la capacité de traitement de la colonne de distillation en place.

Des simulations effectuées sur le logiciel commercial (PROII) ont permis de définir les nouvelles limites d'engorgements de la colonne à distiller dans le procédé selon l'invention, et donc d'accéder à la capacité maximale de traitement du procédé selon l'invention.

Les calculs ont montré une capacité de traitement jusqu'à 37% supérieure au cas de référence pour le cas "amont+aval".

Le procédé selon l'invention dans le cas "amont +aval" présente un intérêt particulièrement important dans le cadre d'un dégoulottage du gaz plant.

La réduction sur le coût de fabrication pour cette configuration est évaluée jusqu'à 17% par rapport au cas de référence, comme le montre le tableau 10 ci dessous.

**Tableau 10 : Évaluation du coût de séparation du propylène pour le cas "amont" et le cas "amont+aval".**

| | Cas de référence | Cas amont (*) | Cas amont+aval (*) |
|---|---|---|---|
| débit charge (kg/h) | 16700 | 19300 | 22900 |
| capacité additionnelle au niveau de la colonne | | 16% | 37% |
| Coût de fabrication / tonne de propylène PG (€/tonne)* | 34,3 | 30,8 | 28,2 |
| Gain coût de fabrication / référence | | 10% | 17,6% |

| | | | |
|---|---|---|---|
| *calculé avec les coûts des utilités en Europe en 2006 | | | |

### Exemple N°3 : Comparaison des conditions de pervaporation et des conditions supercritiques ( selon l'invention)

Une comparaison a été effectuée entre les conditions en pervaporation et les conditions supercritiques pour le cas amont selon l'invention (selon la figure1) mettant en oeuvre une unité de séparation en amont, elle même constitué de 2 modules membranaires en série.

Le cas amont fonctionnant en conditions de pervaporation correspond aux figures 5 et 5 bis, et le cas amont fonctionnant en conditions supercritiques correspond aux figures 6 et 6 bis.

Le fonctionnement en conditions supercritique permet d'éviter la vaporisation à travers la membrane, rendant ainsi inutile le réchauffeur intermédiaire (noté E2 sur la figure 5).

Concernant le cas supercritique, nous avons ici calculé les deux options pour la réinjection du perméat :
- perméat condensé, pompée et réinjecté sous forme liquide dans la colonne (Cas amont supercritique-1 selon la figure 6)
- perméat injecté directement dans la colonne (Cas amont supercritique-2 selon la figure 6 bis).

**Tableau 11 : Configurations utilisées pour les exemples de comparaison des conditions industrielles et des conditions supercritiques**

| | Cas amont supercritique-1 | Cas amont supercritique-2 | Cas amont pervaporation |
|---|---|---|---|
| P_{charge} | 50 bar | 50 bar | 40 bar |
| T_{charge} | 95°C | 95°C | 83°C |
| ΔP module membranaire | 25 bar (Perméat 20 bar) | 30 bar (Perméat 15 bar) | 25 bar |
| Unité de séparation | une unité de séparation en amont incluant deux modules membranaire (cf. Figure 6) | une unité de séparation en amont incluant deux modules membranaire (cf. Figure 6 bis) | une unité de séparation en amont incluant deux modules membranaire (cf. Figure 5) |
| réinjection du perméat dans la colonne | condensation à 50°C (aéroréfrigérant) | réinjection direct dans la colonne (pas de condensation) | condensation à 30°C (condensateur à eau) |

Le flux de charge (1) est porté à une pression supérieure au point critique (P>50bar), ce qui permet de disposer du perméat à une pression d'environ 25 bar (en gardant la même différence de pression (ΔP) au passage de la membrane). De ce fait, le perméat se trouve à plus haute pression et peut être condensé avec un condenseur à air, noté (C2) sur la figure 6, nécessitant moins d'utilités qu'un condenseur à eau tel que celui du cas de fonctionnement en conditions de pervaporation Pour déterminer l'avantage du régime supercritique, un bilan en utilités a été effectué pour le fonctionnement en conditions de pervaporation et le fonctionnement en conditions supercritiques. Le tableau 12 ci dessous rassemble les résultats.

**Tableau 12 : Comparaison des régimes pervaporation et supercritique, et impact sur le coût de séparation du propylène**

| | Cas de référence | Régime de prvaporatio n | Régime Supercritique-1 | Régime Supercritique-2 |
|---|---|---|---|---|
| coût de fabrication du produit (euros/tonnes) | 34,3 | 31,3 | 30 | 30,2 |
| réduction sur les coûts de fabrication/cas de référence | | 8,7 % | 12,5 % | 12,0 % |

L'utilisation des conditions supercritiques dans un schéma "amont" avec une unité de séparation mettant en oeuvre deux modules membranaires permet une réduction sur les coûts de fabrication d'environ 12% par rapport au cas de référence.

## Revendications

1. Procédé de séparation du propane et du propylène mettant en oeuvre une colonne à distiller et au moins une unité de séparation constituée de plusieurs modules membranaires fonctionnant en série, la ou lesdites unités de séparation étant placées soit en amont, soit en aval, soit en amont et en aval de la colonne à distiller, le ou lesdits modules membranaires fonctionnant avec des membranes polymères de perméabilité en propylène supérieure à 0,1 barrer et de sélectivité propane/propylène supérieure à 5, le ou lesdits modules membranaires fonctionnant
- soit aux conditions de pervaporation suivantes prises en entrée de chaque module membranaire : pression de charge comprise entre 20 et 47 bar et température de charge entre 20°C et 92°C, la ou les dites unités de séparation fonctionnant avec une différence de pression telle que le perméat soit à une pression d'au minimum 10 bar,
- soit aux conditions supercritiques suivantes prises en entrée de chaque module membranaire : pression de charge comprise entre 47 et 100 bar et température de charge entre 92°C et 200°C, la ou les dites unités de séparation fonctionnant avec une différence de pression telle que le perméat soit à une pression d'au minimum 10 bar.

2. Procédé de séparation du propane et du propylène selon la revendication 1, dans lequel, le perméat est réinjecté directement dans la colonne en phase vapeur.

3. Procédé de séparation du propane et du propylène selon la revendication 1, dans lequel, le perméat est directement valorisé en tant que propylène haute pureté.

4. Procédé de séparation du propane et du propylène selon la revendication 1, dans lequel, lorsque le ou les modules membranaires fonctionnent en conditions supercritiques, le perméat est condensé, repompé puis réintroduit en phase liquide dans la colonne.

5. Procédé de séparation du propane et du propylène selon la revendication 1, dans lequel la pression du perméat est égale ou supérieure à la pression de la colonne.

## Patentansprüche

1. Verfahren zur Trennung von Propan und Propylen, wobei eine Destillationskolonne eingesetzt wird sowie mindestens ein Trenneinheit, die aus mehreren Membranmodulen besteht, die in Reihe geschaltet sind, wobei die Trenneinheit(en) entweder stromaufwärts oder stromabwärts oder stromaufwärts und stromabwärts der Destillationskolonne angeordnet sind, wobei das/die Membranmodul(e) mit Polymermembranen betrieben werden, deren Permeabilität für Propylen mehr als 0,1 Barrer beträgt und deren Propan/Propylen-Selektivität höher als 5 ist, wobei das oder die Membranmodul(e) folgendermaßen betrieben werden
- entweder unter den folgenden Pervaporationsbedingungen, deren Messung eingangsseitig der jeweiligen Membranmodule erfolgt: Beladungsdruck im Bereich von 20 bis 47 bar und Beladungstemperatur zwischen 20 °C und 92 °C, wobei die Trenneinheit(en) mit einem derartigen Druckunterschied betrieben werden, dass das Permeat unter einem Druck von mindestens 10 bar steht,
- oder unter den folgenden überkritischen Bedingungen, deren Messung eingangsseitig der jeweiligen Membranmodule erfolgt: Beladungsdruck im Bereich von 47 bis 100 bar und Beladungstemperatur zwischen 92 °C und 200 °C, wobei die Trenneinheit(en) mit einem derartigen Druckunterschied betrieben werden, dass das Permeat unter einem Druck von mindestens 10 bar steht.

2. Verfahren zur Trennung von Propan und Propylen nach Anspruch 1, wobei das Permeat als Dampfphase direkt in die Kolonne zurückgeleitet wird.

3. Verfahren zur Trennung von Propan und Propylen nach Anspruch 1, wobei das Permeat direkt als hochreines Propylen verwertet wird.

4. Verfahren zur Trennung von Propan und Propylen nach Anspruch 1, wobei das Permeat kondensiert, zurückgepumpt und dann als Flüssigphase in den Kolonne zurückgeleitet wird, wenn das/die Module unter überkritischen Bedingungen betrieben wird/werden.

5. Verfahren zur Trennung von Propan und Propylen nach Anspruch 1, wobei der Druck des Permeats mindestens so hoch wie der Druck der Kolonne ist.

## Claims

1. Process for separating propane and propylene using a distillation column and at least one separation unit constituted by several membrane modules operating in series, said separation unit or units being placed either upstream, or downstream, or upstream and downstream of the distillation column, said membrane module or modules operating with polymer membranes having a propylene permeability greater than 0.1 barrer and propane/propylene selectivity greater than 5, said membrane module or modules operating
- either under the following pervaporation conditions recorded at the inlet of each membrane module: feedstock pressure comprised between 20 and 47 bar and feedstock temperature between 20°C and 92°C, said separation unit or units operating with a difference in pressure such that the permeate is at a pressure of 10 bar minimum,
- either under the following supercritical conditions recorded at the inlet of each membrane module: feedstock pressure comprised between 47 and 100 bar and feedstock temperature between 92°C and 200°C, said separation unit or units operating with a difference in pressure such that the permeate is at a pressure of 10 bar minimum.

2. Process for separating propane and propylene according to claim 1 in which the permeate is reinjected directly into the column in vapour phase.

3. Process for separating propane and propylene according to claim 1 in which the permeate is directly valorized as high-purity propylene.

4. Process for separating propane and propylene according to claim 1 in which when the membrane module or modules operate under supercritical conditions, the permeate is condensed, repumped and reintroduced in liquid phase into the column.

5. Process for separating propane and propylene according to claim 1, in which the pressure of the permeate is equal to or greater than the pressure of the column.
